# EUROPEAN PATENT APPLICATION

(11) **EP 4 039 789 A1**
(43) Date of publication of application: **10.08.2022**
(21) Application number: 20871602.7
(22) Date of filing: 28.09.2020
(51) Int. Cl.: C12M 1/34, C12Q 1/04, G01N 33/53

(54) **METHOD FOR SCREENING SECRETION-PRODUCING CELLS AND KIT FOR SCREENING SECRETION-PRODUCING CELLS**

(30) Priority: 30.09.2019 JP 2019178577
(71) Applicant: TOKYO OHKA KOGYO CO., LTD., Kawasaki-shi, Kanagawa 211 0012 (JP)
(72) Inventor: OHSAKA Takashi, Kawasaki-shi, Kanagawa 211-0012 (JP)
(74) Representative: Plasseraud IP
(86) International application number: PCT/JP2020/036534
(87) International publication number: WO 2021/065766

(57) **Abstract**

A method for screening secretion-producing cells is provided, in which a cell that produces a secretion of interest is subjected to screening from a plurality of cells. The method includes a step A of capturing the cell and at least one detection particle that is allowed to capture the secretion of interest in each of a plurality of wells having a bottom section with a through-hole sized such that the cell does not pass through, a step B of causing the cell captured in each of the plurality of wells to produce a secretion, a step C of detecting the secretion of interest captured by the at least one detection particle, and a step D of identifying, based on a result of the detection as an index, a well in which a cell producing the secretion of interest is captured from the plurality of wells. Each of the wells has a size allowing the cell to be captured in one cell unit in a state in which one or more detection particles are captured.

## Description

### [Technical Field]

The present invention relates to a method for screening secretion-producing cells and a kit for screening secretion-producing cells.

Priority is claimed on Japanese Patent Application No. 2019-178577, filed September 30, 2019, the content of which is incorporated herein by reference.

### [Background Art]

In recent years, especially in the field of drug discovery, an analytical target of cells has been subdivided from the cell group level to the single cell level. Attempts have been made to capture, identify, select, recover, culture, carry out genetic analysis, and use the selected cells at the individual level. As a method for identifying and selecting cells, a method for separating a secretion secreted from cells, detecting a secretion of interest, and screening a cell secreting the secretion of interest has been adopted. In such single cell analysis, a technique for collectively analyzing a plurality of cells is useful.

As a technique for collectively analyzing cells, for example, a method of spotting a mixture of antibody-producing cells, antigen-binding beads, and fluorescently labeled anti-antibody antibodies on a slide glass, carrying out incubation, and locally increasing levels of fluorescence surrounding the antibody-producing cells to identify B cells that produce target antibodies has been reported (Patent Document 1).

### [Citation List]

### [Patent Document]

[Patent Document 1]
Japanese Patent No. 4603894

### [Summary of Invention]

### [Technical Problem]

However, in the method described in Patent Document 1, since the antibody-producing cells are not immobilized, the antibody-producing cells may move in the culture operation, the fluorescence detection operation, the cell collection operation, or the like. Therefore, accurate screening may not be possible.

The present invention has been made in view of the above circumstance, and an object is to provide a method for screening secretion-producing cells and a kit for screening the secretion-producing cells, by which a cell producing a secretion of interest can be accurately obtained.

### [Solution to Problem]

In order to solve the above problem, the present invention has adopted the following configuration.

A first aspect of the present invention is a method for screening secretion-producing cells, in which a cell that produces a secretion of interest is subjected to screening from a plurality of cells. The method includes a step A of capturing the cell and at least one detection particle in each of a plurality of wells having a bottom section with a through-hole sized such that the cell does not pass through, the at least one detection particle being enabled to capture the secretion of interest and sized not to pass through the through-hole, a step B of causing the cell captured in each of the plurality of wells to produce a secretion, a step C of detecting the secretion of interest captured by the at least one detection particle, and a step D of identifying, based on a result of the detection as an index, a well in which a cell producing the secretion of interest is captured from the plurality of wells, in which each of the wells has a size allowing the cell to be captured in one cell unit in a state in which one or more detection particles are captured.

A second aspect of the present invention is a kit for screening secretion-producing cells, in which a cell that produces a secretion of interest is subjected to screening from a plurality of cells. The kit includes a device including a plurality of wells, and at least one carrier particle, in which each of the wells has a size allowing the cell to be captured in one cell unit in a state in which one or more carrier particles are captured, and has a bottom section with a through-hole sized such that the cell does not pass through, and the at least one carrier particle is sized not to pass through the through-hole.

### [Advantageous Effects of Invention]

According to the present invention, it is possible to provide a method for screening secretion-producing cells and a kit for screening the secretion-producing cells, by which a cell producing a secretion of interest can be accurately obtained.

### [Brief Description of Drawings]

Fig. 1 is a diagram showing an example of a step A in a method for screening secretion-producing cells.
Fig. 2 is a diagram showing an example of a step B in the method for screening secretion-producing cells.
Fig. 3 is a diagram showing an example of a step C in the method for screening secretion-producing cells.
Fig. 4 is a diagram showing an example of a step D in the method for screening secretion-producing cells.
Fig. 5 is a diagram showing a modified example of the step A in the method for screening secretion-producing cells.
Fig. 6 is a diagram showing an example of the method for screening secretion-producing cells in a case in which a detection particle is a cell.
Fig. 7A is a diagram showing an example of a washing step in the method for screening secretion-producing cells.
Fig. 7B is a diagram showing an example of the washing step in the method for screening secretion-producing cells.
Fig. 8 is a plan view showing an embodiment of a device included in a screening kit.
Fig. 9 is a front view of the device of the same embodiment.
Fig. 10 is a perspective view showing a normal cross-section of the device of the same embodiment.
Fig. 11 is a front cross-sectional view of the central section of the device of the same embodiment.
Fig. 12 is a side cross-sectional view of the device of the same embodiment.
Fig. 13 is a perspective view showing an assembly method of the same embodiment of the device of the same embodiment.
Fig. 14 shows fluorescence micrographs of wells of an example to which the screening method according to the embodiment is applied. Fig. 14(A) is a fluorescence micrograph of wells that are not subjected to the washing step. Fig. 14(B) is a fluorescence micrograph of a well subjected to the washing step.

### [Description of Embodiments]

Hereinafter, embodiments of the present invention will be described in detail with reference to the drawings, as the case may be. In the drawings, the same or corresponding parts are designated by the same or corresponding reference numerals, and duplicate descriptions will not be repeated. The dimensional ratio in each figure is exaggerated for the sake of explanation and does not necessarily match the actual dimensional ratio.

### (Method for Screening Secretion-producing Cells)

A first aspect of the present invention is a method for screening secretion-producing cells, in which a cell that produces a secretion of interest is subjected to screening from a plurality of cells. The screening method includes a step A of capturing the cell and at least one detection particle in each of a plurality of wells having a bottom section with a through-hole sized such that the cell does not pass through, in which the at least one detection particle is enabled to capture the secretion of interest and sized not to pass through the through-hole, a step B of causing the cell captured in each of the plurality of wells to produce a secretion, a step C of detecting the secretion of interest captured by the at least one detection particle, and a step D of identifying, based on a result of the detection as an index, a well in which a cell producing the secretion of interest is captured from the plurality of wells. Each of the wells has a size allowing the cell to be captured in one cell unit in a state in which one or more detection particles are captured.

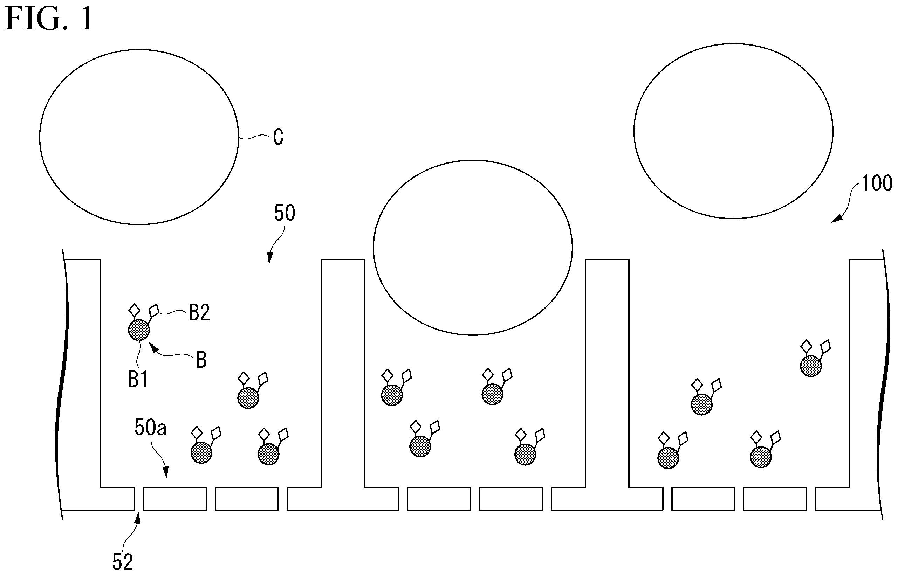

In the screening method according to the present embodiment, first, a cell C and at least one detection particle B capable of capturing a secretion of interest are captured in each of a plurality of wells 50 having a bottom section with at least one through-hole 52 sized such that the cell C to be screened does not pass through (step A; Fig. 1). The at least one detection particle B is sized not to pass through the through-hole 52. Each of the wells 50 has a size allowing the cell C to be captured in one cell unit in a state in which one or more detection particles B are captured.

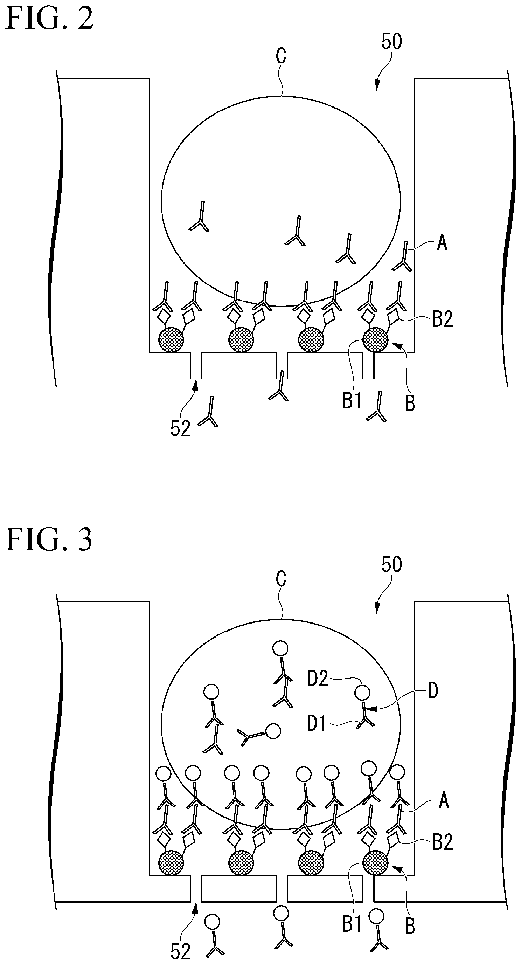

Next, the cell C captured in each of the wells 50 is incubated to produce a secretion A (step B; Fig. 2).

Next, the secretion A of interest captured by the at least one detection particle B is detected using at least one detection reagent D or the like (step C; Fig. 3).

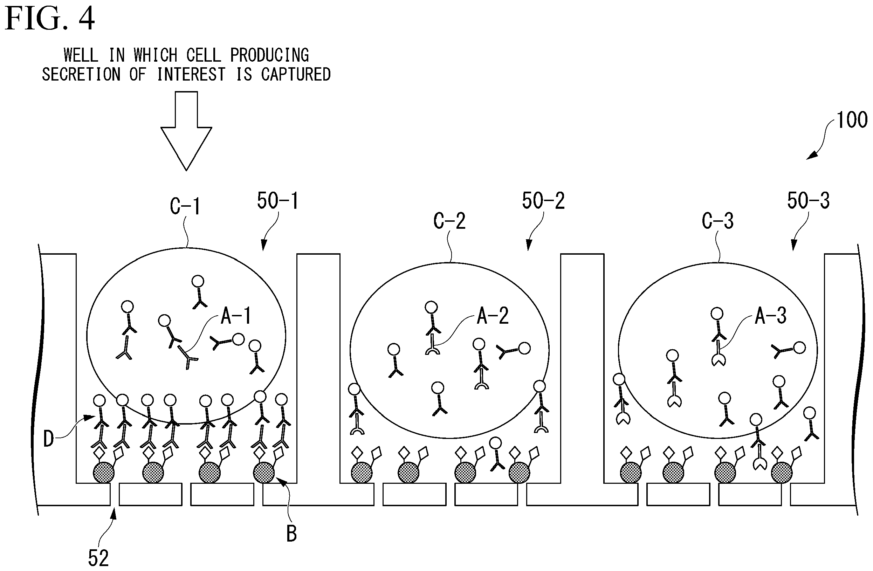

Finally, based on the result of the detection obtained in the step C as an index, the well 50 (well 50-1) in which the cell producing the secretion A (secretion A-1) of interest is captured is identified from the plurality of wells 50 (wells 50-1, 50-2, and 50-3) (step D; Fig. 4).

### <Screening Cell>

A plurality of cells to be screened by the screening method (hereinafter, also referred to as "screening cells") according to the present embodiment is referred to as a cell population predicted to contain cells producing the secretion of interest. The plurality of cells are preferably a population of cells that produce secretions. Examples of cells include mammalian (such as human, mouse, rat, rabbit, horse, camel, and monkey) cells, bird (such as chicken) cells, insect (such as bug) cells, and other animal cells; plant cells; fungus such as yeast; bacteria such as Escherichia coli; and the like. The screening cells may be gene recombinant cells in which genes encoding a secreted protein or a secreted peptide are introduced into these cells. The secreted protein and the secreted peptide may be a chimeric protein in which the secretion signal protein is linked to a non-secretion protein or a non-secretion peptide.

The secretion of interest (hereinafter, also referred to as "interest secretion") may be natural secretions or may be non-natural secretions obtained using genetic engineering. The interest secretion is not limited thereto, and is preferably a single secretion. Examples of the interest secretion include immunoglobulins (immunoglobulin G (IgG), immunoglobulin M (IgM), and the like); interleukins (IL-2, IL-7, IL-12, IL-15, and the like), cytokines such as chemokine, interferons (IFN-γ and the like), hemopoietic factors (a colony stimulating factor, a granulocyte colony stimulating factor, erythropoietin, and the like), cell growth factors (an epithelial growth factor, a fibroblast growth factor, a platelet-derived growth factor, a hepatocyte growth factor, a transforming growth factor, and the like), cytotoxic factors (a tumor necrosis factor, lymphotoxin, and the like), adipokines (leptin, a tumor necrosis factor, and the like secreted from adipose tissue), neurotrophic factors (nerve growth factor, and the like); antibiotics; metabolites of microorganisms such as a pigment; hormones (a peptide hormone, steroid hormone, a microbial hormone, and the like); chimeric protein of secretion signal peptide and non-secretion protein, and the like, and the present embodiment is not limited thereto. The interest secretion is not limited to a natural protein or a natural peptide, and may be a genetically modified secretion.

In one embodiment, the secretion is preferably an antibody. Examples of the natural antibody include immunoglobulin G (IgG), immunoglobulin M (IgM), and the like. Examples of an unnatural antibody include antibody fragments such as Fab, scFv, and Diabody; single domain antibodies (Single Domain Antibodies, Methods in Molecular Biology Volume, 911, 2012); artificial protein molecules having antibody-like properties (Skrlec k, Strukelj B, Berlec A. Non-immunoglobulin scaffolds: a focus on their targets, Trends Biotechnol., 2015, Apr 27), and the like.

Examples of the secretion-producing cells that produce the interest secretion include antibody-producing cells, cytokine-producing cells, and hormone-secreting cells.

Examples of the antibody molecule-producing cells include, but are not limited to, B cells, hybridomas in which B cells and myeloma cells are fused, and gene recombinant cells obtained by introducing a polynucleotide encoding an antibody molecule into a cell. Examples of the cells into which antibody genes are introduced include animal cells, fungus such as yeast, and bacteria such as Escherichia coli. Specific examples of the cells into which the antibody genes are introduced include NSO cells, CHO cells, COS cells, 293FT cells, and the like.

Examples of the cytokine-producing cells include, but are not limited to, macrophages, B cells, T cells, NK cells, NKT cells, dendritic cells, hepatic Kupffer cells, stromal cells, fibroblasts, vascular endothelial cells, and the like.

Examples of the hormone-secreting cells include, but are not limited to, anterior pituitary cells, somatotropic producing cells, lactotropin-producing cells, thyroid-stimulating hormone-producing cells, gonadotropin-producing cells, corticotropin-producing cells, intermediate pituitary cells, melanocyte-stimulating hormone-secreting cells, oxytocin-secreting cells, vasopressin-secreting cells, serotonin-secreting cells, endorphin-secreting cells, somatostatin-secreting cells, gastrin-secreting cells, secretory-secreting cells, cholecystokinin-secreting cells, insulin-secreting cells, glucagon-secreting cells, bombesin-secreting cells, thyroid cells, thyroid epithelial cells, parafollicular cells, parathyroid cells, parathyroid main cells, anoxic cells, adrenal gland cells, chrome-affinitive cells, steroid hormone (mineralocorticoid or glucocorticoid) -producing cells, testosterone-secreting cells, estrogen-secreting cells, progesterone-secreting cells, juxtaglomerular apparatus cells of the kidney, macula densa cells of the kidney, perivascular polar cells of the kidney, mesangium cells of the kidney, and the like.

Among these, the interest secretion is preferably an antibody, and the secretion-producing cells are preferably antibody-producing cells.

### <Step A>

In the step A, the cell and the at least one detection particle are captured in each of the plurality of wells having a bottom section with a through-hole sized such that the cell does not pass through, the at least one detection particle being enabled to capture the secretion of interest and sized not to pass through the through-hole.

Fig. 1 is a diagram showing an example of the step A. A device 100 includes a plurality of wells 50. Each of the wells 50 has the through-hole 52 in a bottom section 50a. The device 100 may be formed with, for example, a substrate, a membrane, or the like, which has a first surface and a second surface. The plurality of wells 50 may be, for example, recessed sections each of which has an opening section onto the first surface of the substrate or the membrane. The through-hole 52 may be a through-hole penetrating from the first surface to the second surface of the substrate or the membrane. It is preferable that a material of the device 100 not be a material harmful to cells. Examples of the material of the device 100 include glass; general resins such as polyethylene terephthalate (PET), polymethylmethacrylate (PMMA), polycarbonate (PC), polystyrene (PS), cycloolefin polymer (COP), epoxy; and the like.

The planar-shaped opening sections of the wells 50 are not particularly limited as long as screening cells C can be captured in one cell unit. Examples of the planar shape of each of the wells 50 include a circle, an ellipse, a polygon (a quadrangle such as a square, a hexagon, and an octagon), and the like. A regular hexagon is preferable from the viewpoint of increasing the disposition density. The bottom section 50a of each of the wells 50 may have a flat bottom or a rounded bottom. The flat bottom is preferable from the viewpoints of ease of forming the through-hole 52 and increasing the storage amount of the detection particles B.

Each of the wells 50 has a size allowing the screening cells C to be captured in one cell unit in a state in which one or more detection particles B described later are captured. The term "one cell unit" means, for example, a single cell. The size of each of the wells 50 can be appropriately selected according to the size of each of the screening cells C. The size of each of the wells 50 is not limited, but generally, the maximum diameter of a circle entering a well opening section can be about 1 to 100 µm, and a depth can be about 1 to 100 µm. The maximum diameter of the circle entering the well opening section is more preferably 2 to 50 µm, and even more preferably 3 to 25 µm. The depth of each of the wells 50 is more preferably 2 to 70 µm, even more preferably 3 to 50 µm, and particularly preferably 4 to 30 µm.

In relation to the size of each of the screening cells C, the maximum diameter of the circle entering each of the wells 50 in plan view can be about 0.5 to 2 times the maximum diameter of each of the screening cells C, and is preferably 0.8 to 1.9 times. The depth of each of the wells 50 can be a size of about 0.5 to 4 times the maximum diameter of each of the screening cells C, and is more preferably 0.8 to 1.9 times.

The through-hole 52 is provided in the bottom section 50a of each of the wells 50 and communicates an internal space of each of the wells 50 with an external space of each of the wells 50. The through-hole 52 penetrates a member forming the bottom section 50a. A position, shape, size, and the like of the through-hole 52 are not particularly limited as long as each of the screening cells C does not pass through the through-hole 52 and a liquid containing the secretion secreted by the screening cells C can pass through the through-hole 52. Examples of the planar shape of the through-hole 52 include a circle, an ellipse, a polygon (a quadrangle such as a square, a hexagon, and an octagon), and the like. From the viewpoint of ease of formation, a circular shape is preferable.

The size of the through-hole 52 is not limited, but generally, the minimum inner diameter of the through-hole 52 can be 10 nm to 20 µm. The minimum inner diameter of the through-hole 52 is preferably 50 nm to 15 µm, and more preferably 100 nm to 10 µm.

In relation to the size of each of the screening cells C, the minimum inner diameter of the through-hole 52 is preferably 0.5 times or less the maximum diameter of the screening cell C, more preferably 0.1 times or less, and even more preferably 0.05 times or less.

The detection particles B are capable of capturing the secretion of interest. Each of the detection particles B has a size allowing each of the detection particles B to be captured in the well 50 and not to pass through the through-hole 52. The size of each of the detection particles B can be appropriately selected according to the size of each of the wells 50 and the size of the through-hole 52. The particle diameter of each of the detection particles B can be, for example, 50 nm to 80 µm. The particle diameter of each of the detection particles B is, for example, preferably 100 nm to 50 µm, more preferably 500 nm to 30 µm, and even more preferably 1 to 20 µm.

In relation to the size of the through-hole 52, the particle diameter of each of the detection particles B is preferably 1.5 times or more the minimum inner diameter of the through-hole 52, more preferably 2 times or more, and even more preferably 3 times or more.

The number of through-holes 52 is not particularly limited and can be appropriately set according to the size of each of the wells 50 and the size of the through-hole 52. The number of through-holes 52 may be one or more, and may be, for example, in the range of 1 to 100. The number of through-holes 52 is preferably two or more from the viewpoint of the outflow efficiency of the liquid in each of the wells 50. Specific examples of the number of through-holes 52 include, for example, 2 to 10, 2 to 5, 2, or 3.

The position of the through-hole 52 is not particularly limited as long as the through-hole 52 is positioned in the bottom section 50a of each of the wells 50. From the viewpoints of facilitating the storage of each of the screening cells C in each of the wells 50 and the efficiency of the outflow of the liquid in each of the wells 50, it is preferable that one or more through-holes 52 be disposed near the center of the bottom section 50a. In a case in which there are a plurality of the through-holes 52, a disposition of the plurality of the through-holes 52 is not particularly limited. For example, the plurality of the through-holes 52 may be disposed together near the center of the bottom section 50a, or may be disposed randomly or in a grid pattern in the bottom section 50a.

The detection particles B are not particularly limited as long as they can capture the interest secretion. Examples of the detection particles B include carrier particles on which a substance having a binding property to the interest secretion is immobilized, cells containing a cell membrane protein having a binding property to the interest secretion, and the like. In the example of Fig. 1, each of the detection particles B is composed of a carrier particle B 1 on which a substance B2 having a binding property to the interest secretion (hereinafter, also referred to as a "binding substance") is immobilized.

In a case in which each of the detection particles B is composed of the carrier particle B1 and the binding substance B2, a material of the carrier particle B1 is not particularly limited, and a carrier particle commonly used for detecting an antibody or the like can be used. Examples of the carrier particle B1 include, but are not limited to, beads (magnetic beads, resin beads, and the like), hydrogel particles (sodium alginate gel, agarose gel, and the like), metal particles (gold nanoparticles,) and the like.

The binding substance B2 specifically binds to the interest secretion. In a case in which the interest secretion is an antibody, an antigen (antigen peptide, antigen protein, or the like) of the antibody can be used as the interest secretion. In a case in which the interest secretion is a cytokine or hormone, as the binding substance B2, for example, an antibody that binds to the cytokine or hormone can be used.

Immobilization of the binding substance B2 on the carrier particle B 1 can be performed by a known method. Examples of the immobilization method include, but are not limited to, a method using a binding pair such as a biotin-avidin bond, a passive adsorption method, and the like.

In the step A, the screening cells C and the detection particles B are captured in the plurality of the wells 50. In this case, it is preferable that one screening cell C be stored in one well 50. An operation of capturing the screening cell C in the well 50 (hereinafter, also referred to as a "cell-capturing operation") and an operation of capturing the detection particles B (hereinafter, also referred to as a "detection particle-capturing operation") may be performed separately or may be performed at the same time. In a case in which each of the capturing operations is performed separately, the order of the cell-capturing operation and the detection particle-capturing operation is not particularly limited. It is preferable to perform the detection particle-capturing operation first from the viewpoint that the detection particles B can be easily disposed evenly in the well 50.

In a case in which the cell-capturing operation and the detection particle-capturing operation are performed separately, the detection particle-capturing operation is performed such that the detection particles B are suspended in an appropriate liquid such as a buffer solution (PBS, physiological saline, or the like) or a culture medium, and the suspension is supplied to the plurality of wells 50. It is not necessary to perform a charging operation of the suspension for each well 50, and the suspension may be supplied to surfaces of the opening sections of the plurality of wells 50. The detection particles B in the suspension supplied to the wells 50 are stored in each well 50 by their own weight. The detection particles B may be stored in the wells 50 while the liquid in the wells 50 is suctioned from suction holes or the like, which communicate with the through-holes 52. In this case, the detection particles B can be stored in the wells 50 more quickly. The storage amount of the detection particles B in each well 50 can be adjusted by a density of the detection particles B in the suspension. The density of the detection particles B in the suspension is not particularly limited as long as the suspension does not impair the fluidity, and can be appropriately selected according to the size of each of the detection particles B. The density of the detection particles B in the suspension can be generally, for example, about 10⁵ to 10¹² particles/mL.

In a case in which a culture medium is used as the liquid in which the suspension particles are suspended, a culture medium for culturing the cells can be appropriately selected depending on the type of screening cells C. In a case in which the screening cell C is a mammalian cell, examples of the culture medium include a MEM medium, a MEMα medium, and a RPMI medium.

In the present specification, the term "appropriate liquid" means a liquid in which the screening cells C can survive. The appropriate liquid is preferably a liquid in which the screening cells C produce secretions in a case in which the screening cells C are secretion-producing cells. Examples of the appropriate liquid include a buffer solution and a culture medium as described above.

The cell-capturing operation can be performed such that the screening cells C are suspended in an appropriate liquid and the cell suspension is supplied to the plurality of wells 50. It is not necessary to perform a charging operation of each of the screening cells C for each well 50, and the cell suspension may be supplied to a surface provided with the opening sections of the plurality of wells 50 on a member (plate, membrane, or the like) including the plurality of wells 50. The screening cells C in the cell suspension supplied to the wells 50 are stored in each well 50 in one cell unit by their own weight. The screening cells C may be stored in the wells 50 while suctioning the liquid in the wells 50 from suction holes or the like, which communicate with the through-holes 52. In this case, the screening cells C can be stored in the wells 50 more quickly. A density of the screening cells C in the cell suspension is not particularly limited as long as the cell suspension does not impair the fluidity, and can be appropriately selected according to the size of each of the screening cells C. The density of the screening cells C in the cell suspension can be generally, for example, about 10⁵ to 10⁹ cells/mL.

In a case in which a culture medium is used as the liquid for suspending cells, the culture medium may be the same as described above.

In a case in which the cell-capturing operation is performed after the detection particle-capturing operation, the detection particle-capturing operation may be performed, the liquid in the wells 50 then may be discharged from the through-holes 52, and thereafter, the cell suspension containing the screening cells C may be supplied to the wells 50. As a result, the excess medium in the wells 50 is removed, so that the efficiency of capturing the screening cells C into the wells 50 can be increased. The liquid in the wells 50 may be discharged while being suctioned from the suction holes or the like, which communicate with the through-holes 52. In this case, the liquid can be efficiently discharged from the through-holes 52.

In a case in which the cell-capturing operation and the detection particle-capturing operation are performed at the same time, the detection particles B and the screening cells C may be suspended in an appropriate liquid, and the suspension may be supplied to the plurality of wells 50. The detection particles B and the screening cells C in the suspension, which are supplied to the wells 50, are stored in each well 50 by their own weight. The detection particles B and the screening cells C may be stored in the wells 50 while suctioning the liquid in the wells 50 from suction holes or the like, which communicate with the through-holes 52. In this case, the detection particles B and the screening cells C can be stored in the wells 50 more quickly. The storage amount of the detection particles B in each well 50 can be adjusted by a density of the detection particles B in the suspension. The density of the detection particles B in the suspension can be the same as described above. The screening cells C are stored in each well 50 in one cell unit. The density of the screening cells C in the cell suspension can be the same as described above.

### <Step B>

In the step B, the screening cells captured in the plurality of wells are allowed to produce a secretion.

Fig. 2 is a diagram showing an example of the step B. In a case in which the screening cells C captured in the wells 50 are secretion-producing cells, the screening cells C can be allowed to secrete the secretion A by the incubation of the screening cells C in the wells 50. Incubation conditions can be appropriately set according to the type of screening cells C. In general, incubation conditions such as 25°C to 38°C and 0.03% to 5% CO2 can be used. Specific examples of the incubation conditions include 37°C and 5% CO₂.

The incubation of the screening cells C captured in the wells 50 is performed in a state in which the screening cells C are present in a liquid such as a buffer solution or a culture medium. The inside of each of the wells 50 is preferably filled with the liquid such as the buffer solution or the culture medium.

In a case in which the secretion A secreted from the screening cells C is the interest secretion, the secretion A binds to the binding substance B2 and is captured by the detection particles B. A part of the secretion A moves from the through-holes 52 to the outside of each of the wells 50 together with the liquid (buffer solution, culture medium, or the like) in each of the wells 50.

The incubation time can be sufficient for the secretion A to be captured by the detection particles B. The incubation time may be appropriately set according to the type of screening cells C, and may be, for example, about 0.5 to 24 hours.

### <Step C>

In the step C, the interest secretion captured by the detection particles is detected.

Fig. 3 is a diagram showing an example of the step C. The interest secretion can be detected by using, for example, at least one detection reagent D. The at least one detection reagent D is composed of, for example, a substance (binding substance) D1 that specifically binds to the interest secretion and a labeled substance D2.

As the binding substance D1, for example, an antibody that specifically binds to the interest secretion can be used. The antibody as the binding substance D1 may be a natural antibody or an unnatural antibody (for example, an antibody fragment such as Fab or scFv). A site of the interest secretion to which the binding substance D1 binds is different from a site to which the binding substance B2 binds. For example, in a case in which the interest secretion is an antibody, the binding substance D1 may be an antibody specifically binding to a constant region of the antibody that is the interest secretion.

The labeled substance D2 is a substance generating a signal that can be detected by any detection device or the like. The labeled substance D2 is not particularly limited, and a labeled substance generally used in the biochemical field can be used. Examples of the labeled substance D2 include enzyme labeling, fluorescent labeling, radioactive substances, and the like. Examples of the enzyme labeling include horseradish peroxidase (HRP), alkaline phosphatase (AP), and the like. Examples of the fluorescent labeling include fluorescein such as (carboxyfluorescein (FAM), JOE (6-carboxy-4',5'-dichloro 2',7'-dimethoxyfluorescein), fluorescein isothiocyanate (FITC), tetrachlorofluorescein (TET), and HEX (5'-hexachloro-fluorescein-CE phosphoroamidite); Cy dye-labeled carboxylic acid such as Cy3 and Cy5; AlexaFluor such as Alexa 568 and Alexa 488; and the like. The labeled substance D2 is preferably fluorescent labeling since a signal is easily detected.

The detection reagents D can be brought into contact with the interest secretion A in each of the wells 50 such that the detection reagents D are dissolved in the appropriate liquid (buffer solution, culture medium, or the like) and the detection reagent solution is supplied to each of the wells 50. The liquid in each of the wells 50 may be discharged from the through-holes 52 before the detection reagent solution is supplied to each of the wells 50. The liquid can be discharged in such a manner that the liquid is removed from the channel disposed below the through-holes 52, for example. The liquid may be removed from the channel in such a manner that the liquid is suctioned from the suction holes or the like, which communicate with the channel. By removing the liquid in each of the wells 50 before supplying the detection reagents D, the detection reagents D can be efficiently supplied into each of the wells 50.

The detection reagents D supplied into each of the wells 50 contact the secretion A in each of the wells 50. In a case in which the secretion A is the interest secretion, at least one detection reagent D binds to the secretion A via the binding substance D1. In a case in which the secretion A is the interest secretion, most of the secretion A in each of the wells 50 is captured by the detection particles B. In this case, the detection reagents D are captured by the detection particles B via the secretion A according to the amount of the secretion A captured by the detection particles B. Therefore, by detecting the signal of the labeled substance D2, the secretion A captured by the detection particles B can be detected.

The signal of the labeled substance D2 may be detected by a method according to the type of labeled substance D2. For example, in a case in which the labeled substance D2 is an enzyme-labeled substance, color development can be carried out using the chromogenic substrate of the enzyme, and a color-developing signal can be detected using an optical microscope. In a case in which the labeled substance D2 is a fluorescence-labeled substance, a fluorescent signal can be detected using a fluorescence microscope. In a case in which the labeled substance D2 is a radioactive-labeled substance, a radioactive signal can be detected using an X-ray microscope.

### <Step D>

In the step D, based on a result of the detection as an index obtained in the step C, a well in which a cell producing the interest secretion is captured from the plurality of wells is identified.

Fig. 4 is a diagram showing an example of the step D. At the end of the step C, each of the screening cell C, the detection particles B, the secretion A secreted from each of the screening cells C, and the detection reagents D are present in the plurality of wells 50. However, the abundance of the detection reagents D in the wells 50 differs depending on the type of secretion A.

A well 50-1 contains a screening cell C-1 that secretes a secretion A-1. The secretion A-1 is the interest secretion and is captured by at least one detection particle B. Therefore, at least one detection reagent D is captured by at least one detection particle B via the secretion A-1 that is the interest secretion. As a result, the detection reagents D are accumulated in the well 50-1 according to the amount of the secretion A-1 captured by the detection particles B.

A well 50-2 contains a screening cell C-2 that secretes a secretion A-2. The secretion A-2 is not the interest secretion and is not captured by the detection particles B. Therefore, the detection reagents D flow out from the through-holes 52 together with the liquid in the well 50-2 without being captured by the detection particles B.

A well 50-3 contains a screening cell C-3 that secretes a secretion A-3. The secretion A-3 is not the interest secretion and is not captured by the detection particles B. Therefore, the detection reagents D flow out from the through-holes 52 together with the liquid in the well 50-3 without being captured by the detection particles B.

As described above, since the detection reagents D are captured in the well 50-1 by the detection particles B, the abundance of the detection reagents D in the well is larger than those in the well 50-2 and the well 50-3. Therefore, in a case in which the signal of the labeled substance D2 is detected, the intensity of the detected signal in the well 50-1 is stronger than those in the well 50-2 and the well 50-3. In other words, it can be said that the screening cell C that produces the interest secretion is stored in the well having a higher signal intensity of the labeled substance D2 than the other wells. Therefore, the well having a higher signal intensity of the labeled substance D2 than the other wells can be identified as a well in which a secretion-producing cell producing the interest secretion is captured.

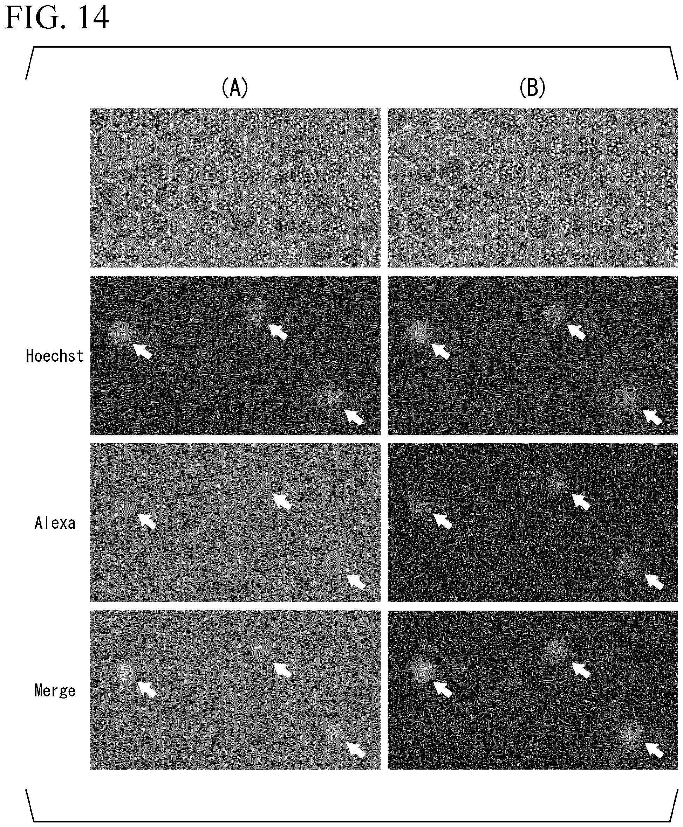

For example, Fig. 14(A) shows fluorescence micrographs of the wells subjected to the steps A to C in Examples. In Fig. 14(A), Alexa 488 is used as the labeled substance D2, and it can be confirmed that fluorescence signals of the wells indicated by the arrows, are stronger than those of the other wells (Fig. 14(A) Alexa). Therefore, the wells indicated by the arrows can be identified as wells in which the secretion-producing cells producing the interest secretion are captured.

### <Modified Example 1>

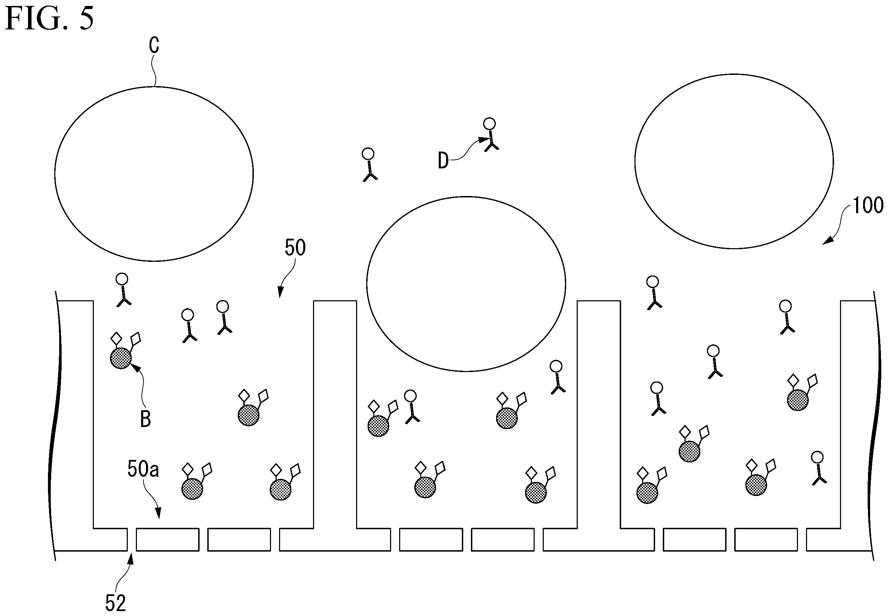

The detection reagents D may be supplied together with the screening cells C and/or the detection particles B to each of the wells 50. Fig. 5 is a diagram showing the step A in a case in which the detection reagents D are supplied together with the screening cells C and the detection particles B to the wells 50.

In the case in which the detection reagents D are supplied together with the screening cells C and the detection particles B to the wells 50, the detection reagents D can be added to a suspension in which the screening cells C and the detection particles B are suspended. Alternatively, the screening cells C and the detection particles B may be suspended in a liquid (buffer solution, culture medium, or the like) to which the detection reagents D are added. Then, the suspension containing the detection reagents D, the screening cells C, and the detection particles B may be supplied to the wells 50.

The detection reagents D can be supplied to the wells 50 while performing either the detection particle-capturing operation or the cell-capturing operation. For example, in a case in which the detection reagents D are supplied to the wells 50 together with the detection particles B, the detection reagents D can be added to an appropriate liquid (buffer solution, culture medium, or the like) in which the detection particles B are suspended. Alternatively, the detection particles B may be suspended in an appropriate liquid (buffer solution, culture medium, or the like) to which the detection reagents D are added. Then, the suspension containing the detection reagents D and the detection particles B may be supplied to the wells 50.

In a case in which the detection reagents D are supplied to the wells 50 together with the screening cells C, the detection reagents D can be added to an appropriate liquid (buffer solution, culture medium, or the like) in which the screening cells C are suspended. Alternatively, the screening cells C may be suspended in an appropriate liquid (buffer solution, culture medium, or the like) to which the detection reagents D are added. Then, the suspension containing the detection reagents D and the screening cells C may be supplied to the wells 50.

In a case in which an operation of discharging the liquid in the wells 50 from the through-holes 52 is performed between the detection particle-capturing operation and the cell-capturing operation, the detection reagents D are preferably added to the suspension used in the capturing operation performed after the discharging operation.

In the step A, the detection reagents D can be supplied to the wells 50 while performing the cell-capturing operation and/or the detection particle-capturing operation, and thereafter the step B can be performed in the same manner as described above. In a case in which the secretion A secreted from each of the screening cells C is the interest secretion, the detection particles B capture the secretion A via the binding substance B2. At least one detection reagent D binds to the secretion A via the binding substance D1. Therefore, in the case in which the secretion A is the interest secretion, the detection reagents D are captured by the detection particles B via the secretion A.

Since the detection reagents D are present in the wells 50 in advance and react with the secretion A, it is not necessary to supply the detection reagents D to the wells 50 in the step C. Therefore, in the step C, the signal of the labeled substance D2 may be detected. The signal of the labeled substance D2 can be detected in the same manner as described above.

The step D can be performed in the same manner as described above, based on the result of the detection obtained in the step C.

### <Modified Example 2>

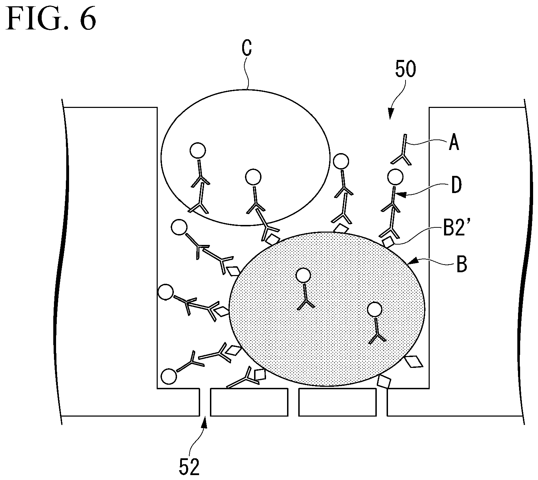

The detection particles B may be cells containing a cell membrane protein having a binding property to the interest secretion. Fig. 6 shows a state of the step C in a case in which the detection particles B are cells (hereinafter, also referred to as "target cells") containing the cell membrane proteins B2' having a binding property to the interest secretion.

The term "membrane proteins" means proteins localized in a cell membrane. The membrane proteins may be integral membrane proteins or peripheral membrane proteins, but are preferably integral membrane proteins. The integral membrane proteins may be multiple-penetrating type proteins that penetrate through a cell membrane multiple times, or may be single-penetrating type proteins that penetrate through a cell membrane once. Examples of the membrane proteins include G protein-coupled receptors (GPCR), ligand-gated ion channels, voltage-gated ion channels, transporters, and the like.

The target cells may be natural cells or gene recombinant cells into which membrane protein genes are introduced. The interest secretion binds to a region where the membrane proteins B2' are exposed on a surface of the cell membrane. In a case in which the interest secretion is an antibody against a membrane protein specific to a diseased cell (for example, a cancer cell), the diseased cells can be used as the target cells.

Each of the target cells has a size that allows the target cells to be captured in each of the wells 50 and not to pass through the through-holes 52. The size of each of the target cells is, for example, about 1 µm to 80 µm. In a case in which the target cells are used as the detection particles B, a size of each of the wells 50 can be set based on the total size of each of the screening cells C and the target cell.

In a case in which a size of the target cell is larger than a size of each of the screening cells C, the maximum diameter of the circle entering the opening section of each of the wells 50 can be, for example, larger than the maximum diameter of the target cell and smaller than twice the maximum diameter of the screening cell C. The depth of each of the wells 50 can be set to have a size about 0.5 to 2 times the total value of the maximum diameter of the target cell and the maximum diameter of each of the screening cells C, is more preferably set to 0.8 to 1.9 times, and even more preferably set to 0.9 to 1.5 times.

In a case in which the target cell is smaller than each of the screening cells C, the size of each of the wells 50 can be set as described above for the detection particles B.

In the step A, the operation of capturing the target cells in the wells 50 (hereinafter, also referred to as a "target cell-capturing operation") may be performed while performing the cell-capturing operation or may be performed separately, but the operation of capturing the target cells and the cell-capturing operation are preferably performed separately. In a case in which the target cell is larger than each of the screening cells C, the target cell-capturing operation is preferably performed prior to the cell-capturing operation.

The target cell-capturing operation can be performed such that the target cells are suspended in an appropriate liquid (buffer solution, culture medium, or the like) and the target cell suspension is supplied to the plurality of wells 50. The target cells C in the target cell suspension supplied to the wells 50 are stored in each well 50 by their own weight. A density of the target cells in the target cell suspension is not particularly limited as long as the target cell suspension does not impair the fluidity, and can be appropriately selected according to the size of each of the target cells. The density of the target cells in the target cell suspension can be generally, for example, about 10⁵ to 10⁹ cells/mL.

The steps B to D can be performed in the same manner as described above. In a case in which the secretion A secreted from each of the screening cells C is the interest secretion, the secretion A binds to the cell membrane protein B2' of the target cell as the detection particles B. In addition, at least one detection reagent D binds to the secretion A. Therefore, the at least one detection reagent D is captured by the cell membrane protein B2'of the target cell via the secretion A. Accordingly, based on the result of the detection of the signal of the labeled substance D2, the well having a higher signal intensity than the other wells can be identified as a well in which the secretion-producing cell producing the interest secretion is captured.

According to the screening method of the present embodiment described above, since the screening cells are stored in the wells in one cell unit and secretion assay is performed, the cells do not move during an assay operation. In addition, since the interest secretion is detected in each well using the detection particles, the distance and time until the interest secretion secreted from the screening cell is captured by the detection particles are shortened. Therefore, as compared with a case in which the interest secretion is detected outside the wells, the diffusion of the interest secretion is suppressed, and the interest secretion can be reliably captured by the detection particles. Therefore, it is possible to accurately identify and obtain the cell that produces the interest secretion.

In addition, since each of the wells 50 has the through-holes 52, the detection reagents that are not captured by the detection particles that flow out from the through-holes 52 together with the liquid in each of the wells 50. Therefore, based on the difference in the signal intensity of the labeled substance D2, the secretion-producing cell that produces the interest secretion can be identified.

### <Other Step>

The screening method of the present embodiment may include another step in addition to the above steps A to D. Examples of other steps include, but are not limited to, a washing step (step E), a secretion-producing cell recovery step (step F), and the like.

### <<Washing Step (Step E)>>

In the washing step, the liquid in each of the wells 50 is discharged from the through-holes 52. Thereafter, an appropriate liquid (buffer solution, culture medium, or the like) is supplied to each of the wells 50 to replace the liquid in each of the wells 50.

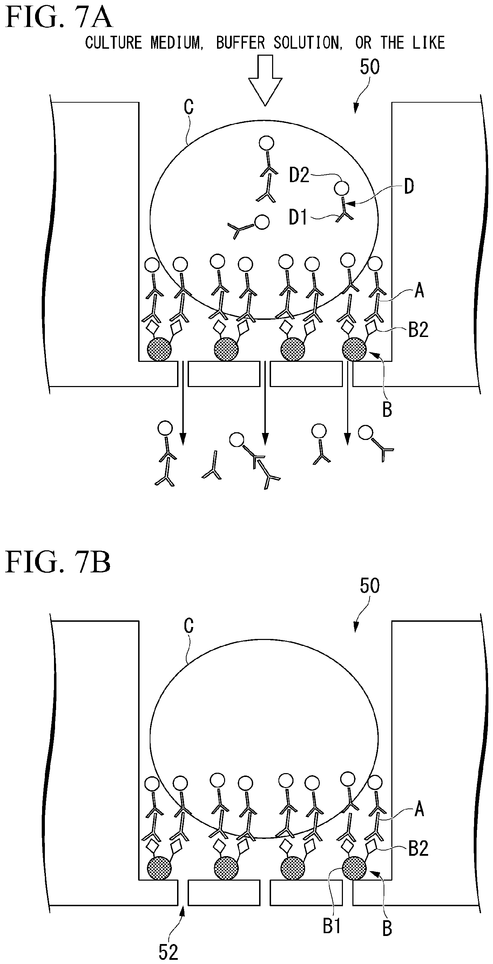

Figs. 7A and 7B are diagrams showing an example of a washing step. Fig. 7A shows an example in which a washing step is performed after the step C. Fig. 7B shows a state after the washing step.

After the step C, in a case in which the secretion A secreted from each of the screening cells C is the interest secretion, most of the secretion A is captured by the detection particles B, and the detection reagents D are captured by the detection particles B via the secretion A. However, it is believed that a part of the detection reagents D remain free (in a state in which another part binds to the secretion A) and are present in each of the wells 50. In a case in which the secretion A is not the interest secretion, all of the detection reagents D in each of the wells 50 remain free in each of the wells 50. In this state, in a case in which the liquid in each of the wells 50 is discharged from the through-holes 52, the free detection reagents D are also discharged out of each of the wells 50 together with the liquid. As a result, the detection reagents D that are freely present in each of the wells 50 can be removed.

Thereafter, an appropriate liquid (buffer solution, culture medium, or the like) is supplied to each of the wells 50 to prevent the screening cells C from drying out.

By performing the washing step after the step C, as shown in Fig. 7B, there are almost no free detection reagents D (detection reagents D that are not captured by the detection particles B) in each of the wells 50. In the wells 50 in which the secretion A secreted from each of the screening cells C is not the interest secretion (for example, the wells 50-2 and 50-3 in Fig. 4), the detection reagent D is almost absent in the wells 50. Therefore, the difference in the signal intensity of the labeled substance D2 between a well in which the secretion A is the interest secretion and another well becomes clearer.

For example, Fig. 14(B) shows fluorescence micrographs of the wells subjected to the washing step after the steps A to C in Examples. In Fig. 14(B), it can be confirmed that the fluorescence signals in wells other than wells indicated by the arrows have almost disappeared. As a result, the wells in which the secretion-producing cells producing the interest secretion are captured can be identified more clearly than those of Fig. 14(A).

In the washing step, the discharge of the liquid in each of the wells 50 and the supply of the liquid in each of the wells 50 may be performed a plurality of times. By discharging and supplying the liquid a plurality of times, it is possible to almost completely remove the free detection reagents D remaining in each of the wells 50.

The washing step may be performed after the step B. By performing the washing step after the step B, the free secretion A that is not captured by the detection particles B can be removed from each of the wells 50. As a result, in a case in which the detection reagents D are supplied to each of the wells 50, the amount of the detection reagents D that react with the free secretion A is decreased, so that the amount of the detection reagents D used can be reduced.

The liquid can be discharged from the through-holes 52 in such a manner that the liquid is removed from the channel disposed below the through-holes 52. For example, in a case in which the channel is filled with the liquid, the wells 50 are also maintained in a state of being filled with the liquid. Here, in a case in which the liquid is removed from the channel, the liquid in each of the wells 50 flows out from the through-holes 52 due to gravity, and most of the liquid in each of the wells 50 can be finally removed. The liquid may be removed from the channel in such a manner that the liquid is suctioned from the suction holes that communicate with the channel.

### <<Secretion-producing cell Recovery Step (Step F)>>

In the secretion-producing cell recovery step, the screening cells C are recovered from the wells identified by the step D as secretion-producing cells that produce the interest secretion. The screening cells C can be recovered using a known single cell recovery means. Examples of the single cell recovery means include a method of recovering cells using a manipulator, a microcapillary, a micropipette, or the like.

### (Kit for Screening Secretion-producing Cell)

A second aspect of the present invention is a kit for screening secretion-producing cells, in which a cell that produces a secretion of interest is subjected to screening from a plurality of cells. The screening kit is provided with a device including a plurality of wells and at least one carrier particle. Each of the wells has a size allowing the cell to be captured in one cell unit in a state in which one or more carrier particles are captured, and has a bottom section with a through-hole sized such that the cell does not pass through. The at least one carrier particle is sized not to pass through the through-hole.

The screening kit according to the present embodiment can be used for the screening method according to the first aspect.

### <Device>

The device includes the plurality of wells with through-holes in the bottom section. Examples of the plurality of wells and the through-holes are the same as those described in the above section (Method for Screening Secretion-producing Cell). Examples of the device including the plurality of wells include those having a configuration as shown in the device 100 of Fig. 1.

The device preferably has a channel that is connected to the through-holes and is disposed below the through-holes. The channel disposed below the through-holes is preferably a channel provided along the outer bottom surface of the plurality of wells. By providing the channel below the through-holes, the liquid in each of the wells can be discharged through the channel and the through-holes. In addition, by providing suction holes communicating with the channel, the liquid in each of the wells can be discharged more smoothly by suction of the liquid from the suction holes.

### <<Specific Example of Device>>

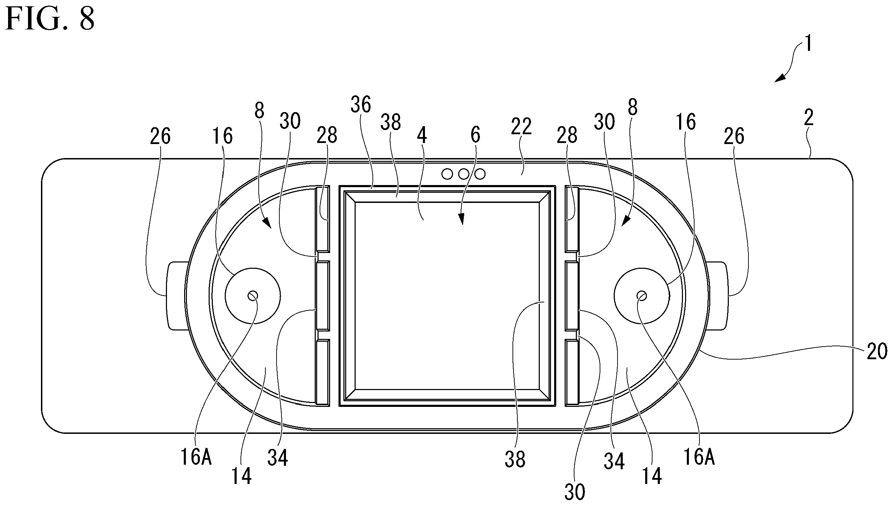

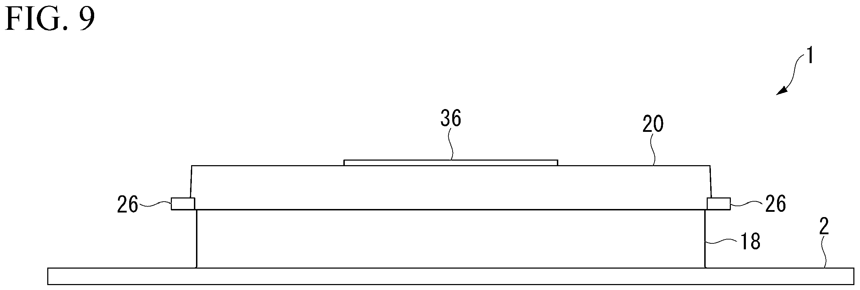

Figs. 8 to 13 show an example of a device having the above configuration. A device 1 has a bottom plate section 2, a cell-mounting membrane (cell-mounting section) 6 provided on the bottom plate section 2 and constituting a cell-mounting surface 4, and a pair of liquid inflow and outflow sections 8 separately provided from the cell-mounting membrane 6 on the bottom plate section 2, and a channel 10 that is provided between the bottom plate section 2 and the cell-mounting membrane 6 and that has channel end sections 12 extending to the pair of the liquid inflow and outflow sections 8. The cell-mounting surface 4 of the cell-mounting membrane 6 is formed with a plurality of wells 50 and through-holes 52 each of which leads from an inner bottom surface of each of the wells 50 to the channel 10. Furthermore, on a back side of each lid section 14, that is, on a ceiling surface of each of the channel end sections 12, a bubble discharge surface 17 that protuberates as in an inclined surface as the bubble discharge surface approaches the suction hole 16A is formed.

The bottom plate section 2 has a constant thickness and is formed in an elongated rectangular plate shape, and the four corners are chamfered round. A projection 2B having a constant height along the outer peripheral edge of the bottom surface over the entire circumference is formed on the bottom plate section 2. A shape of the bottom plate section 2 is not limited to the shape shown in the figure, and may be any shape such as a disk shape, an elliptical shape, or a square shape, and the thickness of the bottom plate section 2 may not be constant as long as a horizontal channel 10 can be formed. In a case where the steps C and D of the screening method according to the first aspect are performed using a microscope, the bottom plate section 2 preferably has a shape that allows the bottom plate section to be placed on a stage of the microscope (for example, a shape similar to a slide glass). In general, it is desirable that the bottom plate section 2 be made of various plastics that are harmless to cells in terms of molding accuracy and cost, but as necessary, the bottom plate section 2 may be made of any material such as ceramic, glass, or metal. A surface of the bottom plate section 2 may be coated with some kind of coating to mitigate the influence on the cells.

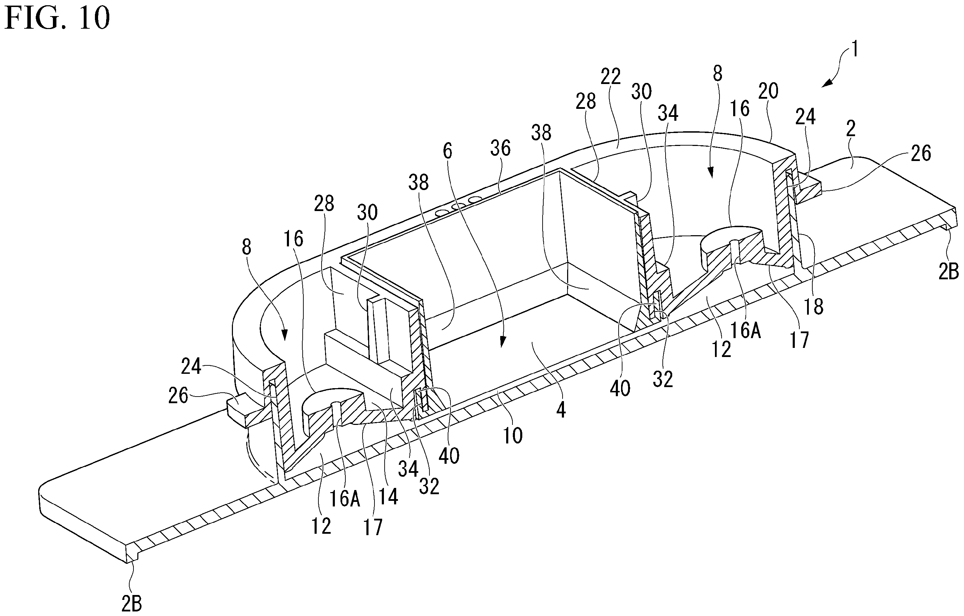

As shown in Fig. 10, on the upper surface of the bottom plate section 2, an engagement wall 18 formed in a rectangular shape and having both semi-circular ends in plan view so as to surround the central section is integrally formed in the state of standing up vertically from the bottom plate section 2. The engagement wall 18 is not limited to the shape shown in the figure, and may be a simple rectangular shape, a circular shape, an elliptical shape, or the like. The height of the engagement wall 18 in this example is uniform over the entire circumference. An outer frame 20 is detachably attached to the engagement wall 18 so as to cover the engagement wall 18 from above over the entire circumference.

The outer frame 20 includes a rectangular peripheral wall section 22 having both semi-circular ends in plan view, and a pair of partition sections 28 provided parallel to each other on the inner peripheral side of the peripheral wall section 22, and the entire is integrally formed. A material of the outer frame 20 is not limited, and in general, may be made of various plastics that are harmless to cells in terms of molding accuracy and cost, but as necessary, the outer frame 20 may be made of any material such as ceramic, glass, or metal. A rectangular parallelepiped space is opened between the partition sections 28, and the cell-mounting membrane 6 is disposed in this space.

The upper end of the peripheral wall section 22 of the outer frame 20 has a cross-sectional shape that is folded outward over the entire circumference, and inside the folded section, a narrow engagement groove 24 that opens downward extends over the entire circumference at a constant depth. The upper end section of the engagement wall 18 is inserted into the engagement groove 24 over the entire circumference and elastically tightened at the folded section of the peripheral wall section 22, so that the outer frame 20 can be detachably fixed to the engagement wall 18. Protrusions 26 that protrude horizontally are each formed at both ends in a longitudinal direction of the outer frame 20, and by lifting these protrusions 26 with a fingertip, the engagement wall 18 can come out from the engagement groove 24, and the bottom plate section 2 and the outer frame 20 can be separated.

The semi-circular regions surrounded by the peripheral wall section 22 and each partition section 28 of the outer frame 20 are each referred to as a liquid inflow and outflow section 8. In this liquid inflow and outflow section 8, lid sections 14 each having a three-dimensional shape in a semi-circular shape in plan view with the center raised upward are formed so as to connect the lower end of the peripheral wall section 22 and the lower end of each partition section 28.

The channel end sections 12 at both ends of the channel 10 are formed between the lid section 14 and the bottom plate section 2, and the lid sections 14 each have a structure that airtightly closes the channel end sections 12. By forming the lid section 14 in this manner, even in a case in which the channel end sections 12 at both ends of the channel 10 are sealed and the device 1 is tilted or shaken, the liquid is prevented from excessively flowing to the left and right through the channel 10 and the channel end sections 12.

In this embodiment, each circular suction hole 16A is formed at substantially the center of each lid section 14, and a cylindrical suction port 16 stands up from each lid section 14 corresponding to the suction hole 16A. A surface of the lid section 14 around the suction port 16 is a liquid storage section, and the liquid overflowing from the suction port 16 is collected. As the liquid storage section, a recessed section may be positively formed around the suction port 16 on the upper surface of the lid section 14.

On the back side of each lid section 14, that is, on the ceiling surface of each of the channel end sections 12, a bubble discharge surface 17 that protuberates as in an inclined surface as the bubble discharge surface approaches the suction hole 16A is formed, and the bubble discharge surface 17 has the suction hole 16A at the apex, to be a conical shape. As a result, in a case in which the channel end sections 12 are filled with a liquid such as a dispersion and contains air bubbles, the air bubbles smoothly move toward the suction hole 16A by buoyancy along the bubble discharge surface 17 and are discharged from the suction hole 16A.

In this embodiment, since a thickness of the lid section 14 is substantially constant, the upper surface of the lid section 14, that is, the upper surface of the liquid storage section is also inclined in a conical shape. Therefore, since the liquid storage section also has a surface that inclines downward as it moves away from the suction port 16 and the liquid spilled from the suction port 16 is collected in the peripheral section of the lid section 14 away from the suction port 16, it is also possible to reduce the possibility of re-inflow from the suction port 16 to cause contamination. However, the present invention is not limited to this configuration, and the upper surface of the lid section 14, that is, the upper surface of the liquid storage section can be made horizontal by increasing the thickness of the lid section 14 as the distance from the suction port 16 increases.

In this embodiment, the suction port 16 stands from the lid section 14, but instead, the standing suction port 16 may not be formed on the lid section 14 and the suction hole 16A may be opened as it is.

At the lower end of each partition section 28 on the liquid inflow and outflow section 8 side, a step section 34 having a constant height from the lower surface is formed over the entire length of each partition section 28, and above the step section 34, each of the two ribs 30 reaching the upper end of each partition section 28 is formed to extend in the vertical direction. The ribs 30 enhance the bending strength of the partition sections 28. Each of the partition sections 28 is formed with an engagement groove 32 that opens on the lower surface of the step section 34 and that has a constant depth, and an engagement projection 42 adjacent to the cell-mounting membrane 6 side of this engagement groove 32.

A square cylindrical frame 36 having a rectangular shape in plan view is detachably housed in a square space surrounded by two linearly extending peripheral wall sections 22 and two partition sections 28. The cell-mounting membrane 6 is stretched over the entire surface of the lower end of the frame 36 with the wells 50 to face upward, and the lower end of the frame 36 and the cell-mounting membrane 6 are bonded without a gap over the entire circumference. The frame 36 is made of a flexible plastic or the like, and in a case in which a force to spread the frame outward is applied, four walls slightly spread outward, tension is applied to the cell-mounting membrane 6, and the loosening of the cell-mounting membrane 6 can be prevented.

Although the thickness of the cell-mounting membrane 6 is not limited, it is preferably about 5 to 100 µm, and more preferably about 10 to 50 µm from the viewpoints of forming the wells 50 capable of capturing the screening cells in one cell unit and forming fine through-holes 52 through which a liquid flows from the bottom of the wells 50 to the back surface side. The cell-mounting membrane 6 may be a multilayer membrane having two or more layers. In that case, a through-hole to be the well 50 is formed in the upper layer, a through-hole to be the through-hole 52 is formed in the lower layer, and these two layers may be bonded together to form the well 50 and the through-hole 52.

A material of the cell-mounting membrane 6 is not limited, and in general, may be made of various plastics that are harmless to cells in terms of molding accuracy and cost, but as necessary, the cell-mounting membrane 6 may be made of any material such as ceramic, inorganic compounds such as poly crystalline or single crystal silicon, glass, or metal. The wells 50 and the through-holes 52 can also be formed by etching or photolithography.

Examples of the planar shape and the size of each of the wells 50 include those in exemplary examples in the above section (Method for Screening Secretion-producing Cell). A plurality of types of the frames 36 including the cell-mounting membrane 6 that has the wells 50 with different sizes may be prepared and combined with the common bottom plate section 2 and the outer frame 20 to form the device 1. Examples of the shape and the size of each of the through-holes 52 include those in exemplary examples in the above section (Method for Screening Secretion-producing Cell).

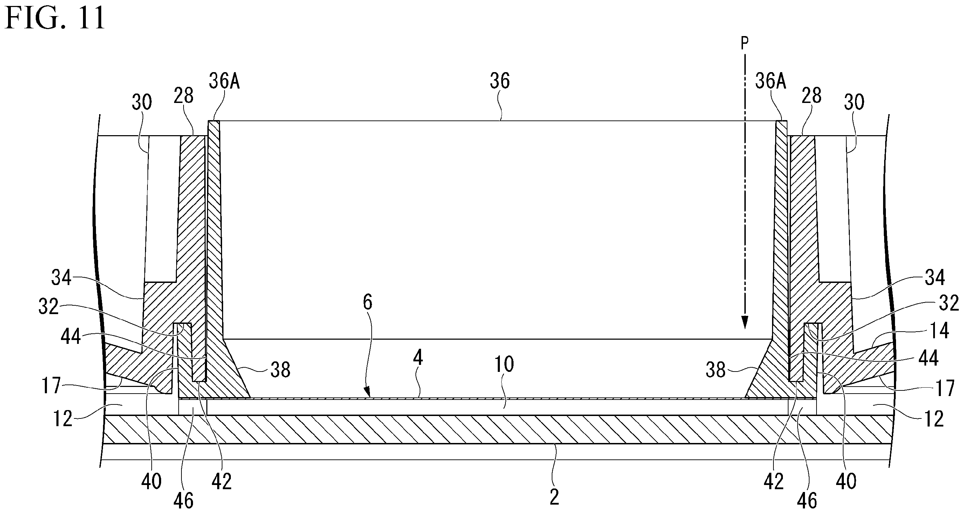

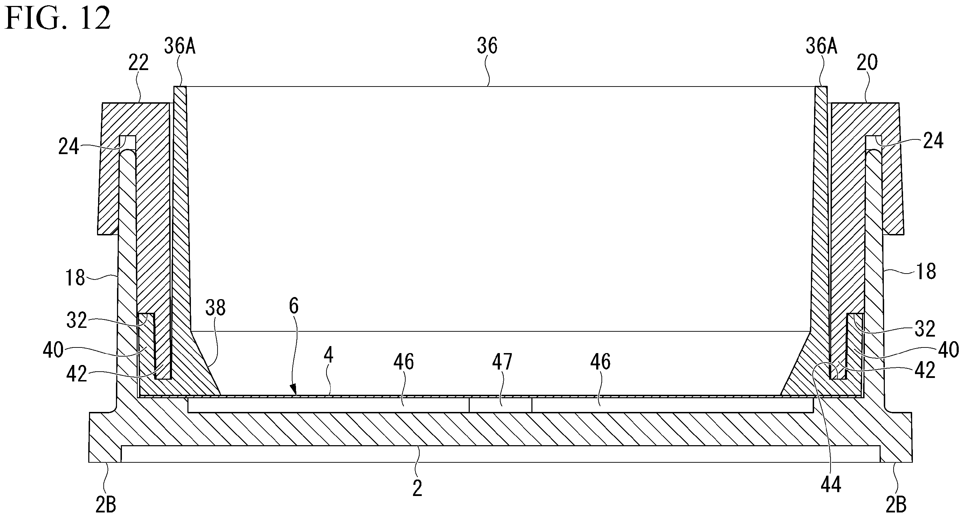

As shown in Figs. 11 and 12, an engagement groove 44 that opens upward and an engagement projection 40 that protrudes downward are formed on the outer peripheral surface of the lower end section of the frame 36 in a shape to be folded upward, over the entire circumference. The depth of the engagement groove 44 and the vertical width of the engagement projection 40 are substantially constant over the entire circumference of the frame 36. The engagement projection 40 is inserted into the engagement groove 32 formed on the lower surface of the outer frame 20, and the engagement projection 42 of the outer frame 20 is inserted into the engagement groove 44 of the frame 36. By these fittings, the frame 36 is fixed to the outer frame 20 in a state in which the frame 36 is housed in the center space of the outer frame 20.

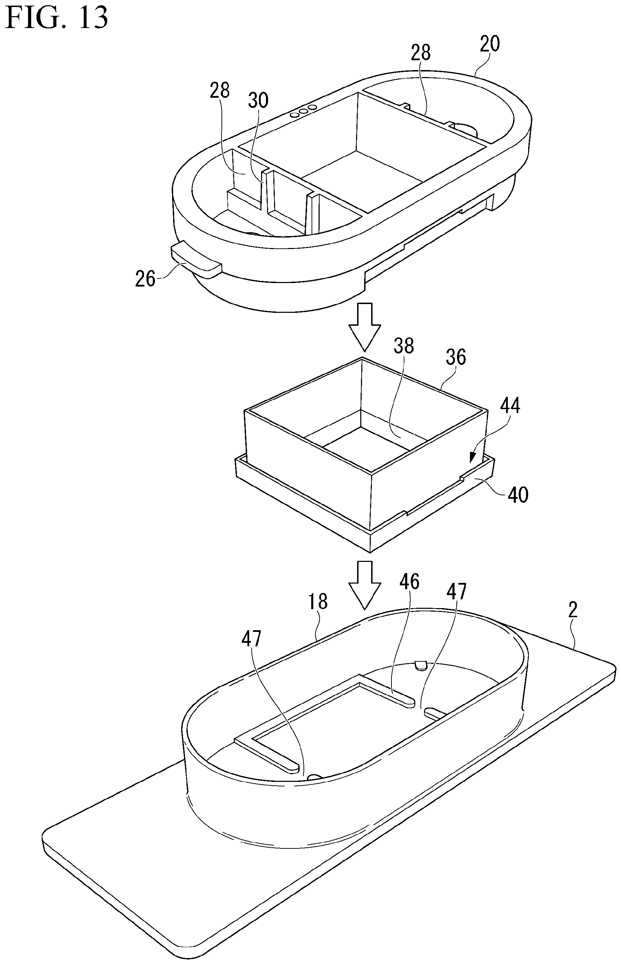

In this case, the lower end surface of the frame 36 abuts on the upper surface of a spacer 46 formed on the bottom plate section 2, and the separation amount of the cell-mounting membrane 6 from the bottom plate section 2, that is, the thickness of the channel 10 is accurately defined according to the thickness of the spacer 46. In this embodiment, as shown in Fig. 13, a pair of U-shaped spacers 46 in plan view are formed inside the engagement wall 18 along the lower end shape of the frame 36, and a notch 47 is formed between the spacers 46. In a state in which the frame 36 is fixed on the bottom plate section 2, the liquid flows from the channel 10 to each channel end section 12 through the notch 47. It not necessary for the spacer 46 to have a shape as shown in the figure, and may abut on the lower surface of the frame 36 at several points. In some cases, the height of the frame 36 from the bottom plate section 2 may be accurately defined by engaging with the outer frame 20 without forming the spacer 46.

An inclined surface 38 having a constant width over the entire circumference is formed on the inner peripheral surface of the lower end section of the frame 36, and the inclined surface 38 protrudes toward the cell-mounting membrane 6 as the inclined surface 38 extends downward. Due to the formation of the inclined surface 38, in a case of recovering secretion-producing cells from the wells 50 of the cell-mounting membrane 6 with an instrument such as a manipulator, a micropipette, or a microcapillary, the cells can be easily recovered even though the wells 50 containing the secretion-producing cells are positioned in front of the inner peripheral edge of the frame 36. The inclined surface 38 also has the effect of increasing an adhesive area of the cell-mounting membrane 6 to the frame 36 and increasing the bonding strength of the cell-mounting membrane 6, and is also useful for increasing the strength of the frame 36.

In a case of manufacturing a cell-screening device having the above configuration, the bottom plate section 2, the frame 36, and the outer frame 20 are separately formed as shown in Fig. 13, and the frame 36 is first formed and fitted onto the lower surface of the outer frame 20, and the outer frame 20 is then formed and fitted to the engagement wall 18 of the bottom plate section 2 to obtain the completed state as shown in Figs. 8 to 12.

In the process of fitting the frame 36 onto the lower surface of the outer frame 20, as shown in Figs. 11 and 12, the engagement projection 42 of the outer frame 20 is fitted into the engagement groove 44 of the frame 36, and the engagement projection 40 of the frame 36 is fitted to the engagement groove 32 of the outer frame 20 to fix each other firmly by the elasticity of each engagement section. At the same time, the inner peripheral surface of the engagement projection 40 and the outer peripheral surface of the engagement projection 42 are shaped to be slightly inclined outward as at least one extends upward. As a result, as the engagement progresses, the engagement projections 40 on the four sides of the frame 36 are pulled outward by the engagement projections 42 of the outer frame 20, and the lower end section of the frame 36 is slightly expanded in four directions, thereby causing tension to pull the four sides of the cell-mounting membrane 6, and as a result, a uniform tension is applied to the cell-mounting membrane 6.

Therefore, even though there is slight loosening in the cell-mounting membrane 6 during the free state of the frame 36, it is advantageous that the loosening in the cell-mounting membrane 6 be eliminated in a case in which the frame 36 is fixed to the outer frame 20, the flatness of the cell-mounting surface 4 of the cell-mounting membrane 6, on which a large number of wells 50 are formed, be increased, the cell-mounting surface 4 be filled with a dispersion in which cells are dispersed, and the cell-screening device be shaken to allow the screening cells to flow evenly, so that the screening cells can be easily captured in the wells 50 in one cell unit.

In this embodiment, the channel end sections 12 positioned at both ends of the channel 10 are closed by the lid sections 14, and the lid sections 14 are provided with the suction ports 16 having the suction holes 16A communicating with the channel 10, so that the lid section 14 suppresses the movement of the liquid in the channel end sections 12 and the channel 10, and the liquid can be taken in and out of the channel 10 through the suction port 16. Therefore, even though the device 1 is carried or tilted in a state in which the screening cells C are captured in the wells 50 of the device 1 and the channel 10 is filled with the liquid, it is difficult for the liquid to move along the channel 10 and the channel end sections 12, which can suppress a so-called sloshing phenomenon. Therefore, it is possible to suppress a problem of the screening cells C and the detection particles B stored in the wells 50 being removed due to part of the liquid flowing into the wells 50 through the through-holes 52.

In this embodiment, even though the liquid overflows from the suction holes 16A of the suction ports 16, the liquid storage section (the peripheral section on the upper surface of the lid section 14) receives the liquid, and the liquid can be suppressed from entering again into the channel 10 from the suction ports 16, so that the risk of contamination from the outside can be reduced, for example. Since the partition section 28 is formed between the cell-mounting surface 4 and the lid section 14, it is possible to suppress the liquid from flowing to the cell-mounting section 6 even though the liquid is collected in the liquid storage section 14.

In this embodiment, since the cell-mounting membrane 6 is positioned at the correct position on the bottom plate section 2 by the lower end of the frame 36 abutting on the spacer 46 of the bottom plate section 2, the flow of liquid to and from the wells 50 and the channel 10 is as desired, and highly accurate screening can be achieved.

In this embodiment, the bottom plate section 2 and the outer frame 20 are formed as separate bodies, and the lid section 14 and the peripheral wall section 22 are accurately disposed at a position with respect to the bottom plate section 2 simply by attaching the outer frame 20 to the bottom plate section 2. Therefore, the device 1 can be easily assembled. In addition, the outer frame 20 can be removed from the bottom plate section 2 after use, and maintenance is easy.

In this embodiment, the cell-mounting membrane 6, the lid section 14, and the peripheral wall section 22 can be accurately positioned simply by forming the bottom plate section 2, the outer frame 20, and the frame 36 as separate bodies, and attaching the frame 36 and the outer frame 20 to the bottom plate section 2, which can improve the ease of assembly. In addition, the frame 36 and the outer frame 20 can be removed from the bottom plate section 2 after use, and maintenance is easy.

In this embodiment, since the cell-mounting section 6, the lid section 14, the peripheral wall section 22, and two suction ports 16 can be accurately positioned simply by forming the bottom plate section 2 and the outer frame 20 as separate bodies, and attaching the outer frame 20 to the bottom plate section 2, assembly is easily achieved.

In the device 1, the screening cells C, the detection particles B, and the detection reagents D are supplied to the wells 50 from above the cell-mounting surface 4 in the cell-mounting membrane 6. The liquid in the wells 50 can be discharged from the through-holes 52. By suction from the suction holes 16A, discharge can be performed more efficiently. The suction from the suction holes 16A may be performed by suctioning the liquid from the suction holes 16A using a micropipette or the like. Alternatively, a suction pump may be connected to the suction port 16 and suction may be performed from the suction holes 16A using the suction pump.

### <Carrier Particles>

The carrier particles are the same as the carrier particles B1 described in the above section (Method for Screening Secretion-producing Cell). The carrier particles may be carrier particles (detection particles B in the first aspect described above) on which a substance having a binding property (binding substance) to the interest secretion is immobilized. The substance having a binding property to the interest secretion is the same as the binding substance B2 described in the above section (Method for Screening Secretion-producing Cell).

The carrier particles may be carrier particles on which the binding substance is not immobilized. In this case, the carrier particles are capable of immobilizing the binding substance. The carrier particles may be surface-modified so that the binding substance can be immobilized, or may be formed of a material capable of immobilizing the binding substance.

For example, in a case in which the binding substance is immobilized using a binding pair such as a biotin-avidin bond, the surface of each of the carrier particles may be modified with one of the binding pair. Specific examples include carrier particles coated with streptavidin.

In a case in which the binding substance is immobilized using the passive adsorption method, the carrier particles may be made of a material having a property of adsorbing the binding substance. In a case in which the binding substance is a peptide or protein, the carrier particles can be polystyrene particles, gold nanoparticles, or the like.

In a case in which the binding substance is not immobilized on the carrier particles, the user can immobilize any binding substance on the carrier particles.

### <Another Configuration>

The screening kit of the present embodiment may include another configuration in addition to the above device and the carrier particles. Another configuration includes at least one detection reagent for the interest secretion, a culture medium or a buffer solution, a labeling reagent for a binding substance, an instruction manual for use, and the like.

The at least one detection reagent for the interest secretion is the same as the at least one detection reagent D described in the above section (Method for Screening Secretion-producing Cell). Examples of the culture medium or the buffer solution are the same as those in exemplary examples in the above section (Method for Screening Secretion-producing Cell). The labeling reagent for the binding substance is used to modify the binding substance so that the binding substance can be immobilized on the carrier particles. For example, in a case in which the carrier particles are streptavidin coated, the labeling reagent includes a biotin labeling reagent.

### <How to use>

The screening kit of the present embodiment can be used to perform the screening method according to the first aspect, and can be used as described in the above section (Method for Screening Secretion-producing Cell).

### [Examples]

Hereinafter, the present invention will be described in more detail with reference to Examples, but the present invention is not limited thereto.

### <Preparation of Cell>

A gene encoding a mouse anti-mouse RANKL monoclonal antibody (hereinafter referred to as a "mouse monoclonal antibody") was introduced into 293FT cells. The transgenic cells were cultured in a MEMα medium supplemented with fetal bovine serum (FBS) for 48 hours and used as cells for a secretion assay.

### <Preparation of Detection Particle>

5 µL of a goat anti-mouse antibody (Jackson ImmunoResearch Inc. Code: 115-005-003) was added to 500 µL of polybead polystyrene microspheres (particle diameter 6.0 µm; 07312, Polysciences, Inc.; hereinafter referred to as "beads") washed with phosphate buffer (PBS) and reacted at 4°C overnight to bind the goat anti-mouse antibody to the beads. The beads were washed with PBS to remove unreacted antibodies. Next, 1% of BSA/PBS was added to the beads, and reacted at 4°C for 1 hour, and thereafter the beads were washed with PBS. This was used as detection particles.

### <Secretion Assay>

### [Step A]

A secretion assay was performed using a device with a plurality of wells with through-holes with a pore size of 2 µm at a bottom section of the device. The detection particles prepared in the above <Preparation of Detection Particle> were added to the wells pre-wet with phosphate buffer (PBS), and the detection particles were spread in each of the wells.

Subsequently, the cell culture solution prepared in the above <Preparation of Cell> was seeded in each of the wells, and the cells were captured in the wells.

### [Step B]

Subsequently, Hoechst 33342 (H342, DOJINDO LABORATORIES.) and Alexa 488-labeled goat anti-mouse IgG antibodies (model "#A11001", Life Technologies LLC) were diluted 500-fold with a MEMα medium with fetal bovine serum (FBS) and added to each of the wells.

The device was left to stand in a CO₂ incubator at 37°C with a CO₂ concentration of 5% for 3 hours with the detection particles in a state in which the cells, and Hoechst 33342 and Alexa 488-labeled goat anti-mouse IgG antibodies were in the wells. According to this method, the cells secrete mouse monoclonal antibodies. The secreted mouse monoclonal antibodies were captured by the goat anti-mouse antibodies of the detection particles, and furthermore, bound to Alexa 488-labeled goat anti-mouse IgG antibodies. As a result, an antigen-antibody complex consisting of goat anti-mouse antibody-mouse monoclonal antibody-Alexa 488-labeled goat anti-mouse IgG antibody was formed.

### [Step C (1)]

The device after leaving to stand in the CO₂ incubator for 3 hours was observed with a fluorescence microscope (model "BZ-9000", manufactured by KEYENCE CORPORATION). Fig. 14(A) is a typical fluorescence micrograph taken from the bottom section side of the wells. As a result, nuclei of the cells were detected by the blue fluorescence of Hoechst 33342 (Hoechst; arrows). In addition, the mouse monoclonal antibodies bound to the detection particles were detected by the green fluorescence of Alexa 488 (Alexa; arrows).

### [Step D (1)]

From the fluorescent signal detected in the step C (1), a well in which the secretion-producing cell secreting the mouse monoclonal antibody was captured could be identified (Merge; arrows).

### [Step E (Washing Step)]

The solution in the well was discharged through the through-holes and PBS was added to the well to wash the detection particles. According to this method, the unreacted Alexa 488-labeled goat anti-mouse IgG antibody was removed.

### [Step C (2)]

The washed device was observed with a fluorescence microscope. Fig. 14(B) is a typical fluorescence micrograph taken from the bottom section side of the wells. As a result, nuclei of the cells were detected by the blue fluorescence of Hoechst 33342 (Hoechst; arrows). In addition, the mouse monoclonal antibodies bound to the detection particles were detected by the green fluorescence of Alexa 488 (Alexa; arrows). In addition, the unreacted Alexa 488-labeled goat anti-mouse IgG antibody was removed by the washing step, and the background was reduced. As a result, fluorescence signals of the mouse monoclonal antibodies captured by the detection particles were clarified.

### [Step D (2)]

From the fluorescent signal detected in the step C (2), a well in which the secretion-producing cell secreting the mouse monoclonal antibody was captured could be identified (Merge; arrows).

From the above results, it was confirmed that the secretion-producing cells that produce the secretion of interest can be screened by the screening method according to the present invention. In addition, by carrying out the washing step, the background was reduced, and the signal of the secretion of interest secreted from the secretion-producing cells could be further clarified.

### [Reference Signs List]

1: Device
2: Bottom plate section
4: Cell-mounting surface
6: Cell-mounting membrane (cell-mounting section)
8: Liquid inflow and outflow section
10: Channel
12: Channel end section
14: Lid section (fluid storage section)
16: Suction port
16A: Suction hole
17: Bubble discharge surface
18: Engagement wall
20: Outer frame
22: Peripheral wall section
24: Engagement groove
26: Protrusion
28: Partition section
30: Rib
32: Engagement groove
34: Step section
36: Frame
36A: Wall section
38: Inclined surface
40: Engagement projection
42: Engagement projection
44: Engagement groove
46: Spacer
47: Notch
50: Well
50a: Bottom section
52: Through-hole
A, A-1, A-2, A-3: Secretion
C, C-1, C-2, C-3: Screening Cell
B: Beads
B1: Carrier particle
B2: Binding substance
B2': Cell membrane protein
D: Detection reagent
D1: Binding substance
D2: Labeled substance

## Claims

1. A method for screening secretion-producing cells, in which a cell that produces a secretion of interest is subjected to screening from a plurality of cells, the method comprising:
a step A of capturing the cell and at least one detection particle in each of a plurality of wells having a bottom section with a through-hole sized such that the cell does not pass through, the at least one detection particle being enabled to capture the secretion of interest and sized not to pass through the through-hole;
a step B of causing the cell captured in each of the plurality of wells to produce a secretion;
a step C of detecting the secretion of interest captured by the at least one detection particle; and
a step D of identifying, based on a result of the detection as an index, a well in which a cell producing the secretion of interest is captured from the plurality of wells,
wherein each of the wells has a size allowing the cell to be captured in one cell unit in a state in which one or more detection particles are captured.

2. The method for screening secretion-producing cells according to Claim 1, wherein the secretion of interest is an antibody.

3. The method for screening secretion-producing cells according to Claim 1 or 2, wherein the detection particle is a carrier particle on which a substance having a binding property to the secretion of interest is immobilized.

4. The method for screening secretion-producing cells according to Claim 1 or 2, wherein the detection particle is a cell containing a cell membrane protein having a binding property to the secretion of interest.

5. A kit for screening secretion-producing cells, in which a cell that produces a secretion of interest is subjected to screening from a plurality of cells, the kit comprising:
a device including a plurality of wells; and
at least one carrier particle,
wherein each of the wells has a size allowing the cell to be captured in one cell unit in a state in which one or more carrier particles are captured, and has a bottom section with a through-hole sized such that the cell does not pass through, and
the at least one carrier particle is sized not to pass through the through-hole.

6. The kit for screening secretion-producing cells according to Claim 5, wherein the device further includes a channel connected to the through-hole and disposed below the through-hole.

7. The kit for screening secretion-producing cells according to Claim 6, wherein the device further includes a suction hole communicating with the channel.

8. The kit for screening secretion-producing cells according to any one of Claims 5 to 7, wherein the secretion is an antibody.

9. The kit for screening secretion-producing cells according to any one of Claims 5 to 8, wherein the carrier particle is a carrier particle on which a substance having a binding property to the secretion of interest is immobilized.

10. The kit for screening secretion-producing cells according to any one of Claims 5 to 8, wherein the carrier particle is a carrier particle allowing a substance that has a binding property to the secretion of interest to be immobilized.
